# EUROPEAN PATENT SPECIFICATION

(11) **EP 1 086 671 B1**
(45) Date of publication and mention of the grant of the patent: **11.05.2005**
(21) Application number: 00305531.6
(22) Date of filing: 30.06.2000
(51) Int. Cl.: A61F 5/01, F16B 7/10

(54) **Orthopaedic brace having a range of motion hinge with an adjustable-length strut**
Orthese mit einem Bewegunsbereich aufweisendem Gelenk und mit längenverstellbaren Stangen
Attelle orthopédique comportant un pivot ayant une plage de mouvement avec une tige de longueur réglable

(30) Priority: 27.09.1999 US 156342 P
(43) Date of publication of application: 28.03.2001
(73) Proprietor: DJ Orthopedics, LLC, Vista, CA 92083-8339 (US)
(72) Inventor: TELLES, Jeffrey Lee, Apartment 42 Tracy CA 95376 (US); ENZERINK, Robert-Jan, Davis CA 95616 (US); FINKES, James Gregory, Patterson CA 95363 (US)
(74) Representative: Belcher, Simon James

(56) References cited:
- EP-A- 0 665 160
- US-A- 3 805 773
- US-A- 4 817 588
- US-A- 4 982 732
- US-A- 5 138 911

## Description

The present application relates to orthopaedic braces adapted with an adjustable-length strut for use in stabilizing a joint after invasive surgery.

In order to ensure the proper healing of a human joint after an injury or invasive surgery, it is often desirable to limit the pivotal motion of the human joint to a predetermined angular range between full extension and full flexion. The pivotal motion may be limited by a range of motion hinge disposed between an upper strut and a lower strut. In order for the orthopaedic brace to function properly, the struts must be adaptable to the body proportions of the patient.

Orthopaedic braces of this general type, including information relating to range of motion braces, and how and why such equipment is used, are disclosed in US-552143, US-649237, US-4776326, US-4817588, US-4982732, US-5052379 and US-5018514.

It is well known that the orthopaedic braces described in the aforementioned patents suffer various problems, shortcomings and disadvantages. In some cases such braces cannot be adjusted to fit the patient, rather, the braces come in various fixed sizes. Alternatively, the braces are not easily adjustable, requiring, for example, tools to change the size of the struts. Some braces require actual cutting or breaking off pieces of the struts to permanently change the length of the struts. Others which rely upon friction to fix the strut at a desired length to not lock the length of the strut positively.

US 3 805 773 discloses a training assist brace having adjustable upper and lower leg members which are pivotally connected to each other. The present invention is an orthopaedic brace that has adjustable length struts.

According to the present invention there is provided an orthopaedic brace comprising:
a first strut;
a second strut;
a hinge disposed between the first and second struts and configured to allow movement of one of the first and second struts about an axis defined by the hinge; and
an adjustment assembly disposed on one of the first and second struts and configured to cooperate with the one of the first and second struts to adjustably set an operative length of the one of the first and second struts;
the one of the first and second struts including a plurality of apertures corresponding to a plurality of strut length settings; characterized in that
the adjustment assembly comprises:
   a body having a slot configured to slidably receive the one of the first and second struts; and
   a retention assembly configured to engage any one of the plurality of apertures to set the length of the one of the first and second struts, the retention assembly including:
an actuator;
a spring;
a fastener; and
a bushing;
in which the fastener couples the bushing to the actuator with the spring disposed between the actuator and the bushing.

In another form, not of the present invention provides an orthopaedic brace including an upper strut, a lower strut, a hinge disposed between the upper strut and the lower strut, and an adjustment assembly disposed on one of the first and second struts. The hinge is configured to allow movement of one of the upper and lower struts about an axis defined by the hinge. One of the upper and lower struts has a plurality of notches defining a plurality of strut length settings. The adjustment assembly is configured to cooperate with any one of the plurality of notches of the one of the first and second struts to selectively set a length of the one of the first and second struts.

In yet another form, not of the present invention provides an orthopaedic brace including an upper strut, a lower strut, a hinge disposed between the upper strut and the lower strut, an upper adjustment assembly disposed on the upper strut, and a lower adjustment assembly disposed on the lower strut. The hinge is configured to allow movement of one of the upper and lower struts about an axis defined by the hinge. The upper adjustment assembly is configured to cooperate with the upper strut to adjustably set a length of the upper strut. The lower adjustment assembly is configured to cooperate with the lower strut to adjustably set a length of the lower strut.

Accordingly, the present invention improves upon the prior art by providing an orthopaedic brace strut that may be changed in length without the use of tools and with the ability to return to the original length, or some other length as desired.

The present invention also provides for a single-action positive lock for a strut length adjustment assembly rather than relying on friction. The ability to size and resize the struts provides a cost-effective and comfortable means to apply an orthopaedic brace to virtually any joint on the human body and eliminates the need to carry large inventories of braces that cannot be sized. By providing a positive lock, the improved brace also better protects the patient and speeds recovery.

The present invention also allows contoured wings, with cushioning material and/or non-slip material attached, to be used to limit movement of the brace after it has been attached and to provide increased comfort to the patient.

The present invention will now be described by way of example with reference to the accompanying drawings, in which:
FIG. 1 is a side perspective view of an adjustable orthopaedic brace assembly having adjustable-length strut assemblies that embodies principles of the present invention showing the brace operatively connected to a human leg;
FIGS. 2A and 2B are, respectively, top and underside perspective views of an adjustable-length strut assembly for the orthopaedic brace of FIG. 1;
FIG. 3 is an exploded, perspective view of the adjustable-length strut assembly of FIGS. 2A and 2B;
FIG. 4 is a cross-sectional view through the adjustable-length strut assembly taken along line 4-4 of FIG. 3;
FIG. 5 is a perspective view of a second embodiment of an adjustable-length strut assembly;
FIG. 6 is a cross-sectional view through the second embodiment of the adjustable-length strut assembly taken along line 6-6 of FIG. 5;
FIG. 7 is a cross-sectional view through the second embodiment of the adjustable-length strut assembly taken along line 7-7 of FIG. 6; and
FIG. 8 is a perspective view of a third embodiment of an adjustable-length strut assembly.

In the drawings, corresponding reference characters indicate corresponding parts throughout the several views.

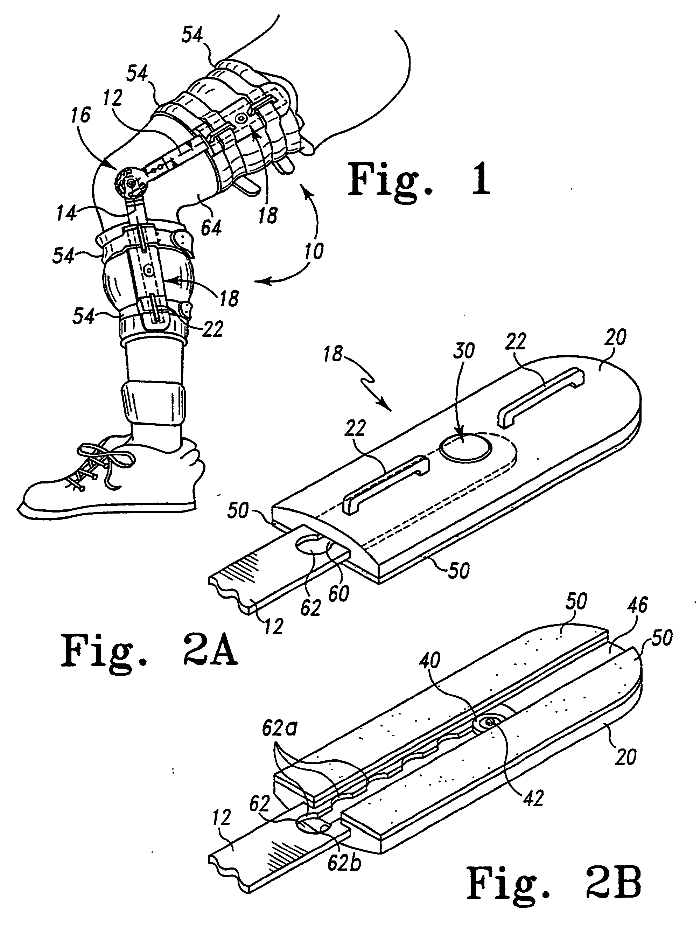

Referring to the drawings, Fig.1 shows an orthopaedic brace 10 operatively attached to a leg 64 using a plurality of straps 54 mounted on an upper strut 12 and a lower strut 14 with a hinge assembly 16 disposed between the upper strut 12 and the lower strut 14. While only one side of the orthopaedic brace 10 is shown (i.e. the hinge assembly 16, the upper strut 12, and the lower strut 14 or "assembly") it should be understood that an identical, but mirror image, assembly is provided on the opposite side of the leg 64.

Each strut 12 and 14 is provided with a preferably identically configured wing assembly 18 although variations in either are contemplated, which is slidably mounted for adjustable movement on the elongated struts 12 and 14. Stated in another manner, each strut 12 and 14 is adjustable in length relative to the length of the strut between the hinge 16 and the straps 54 through adjustable strut assemblies 18. Such will be considered hereafter as the length adjustment of a strut. It should be appreciated that such assemblies 18 may be provided on both struts 12 and 14, or only on one of the two struts 12 and 14. As well, it should also be appreciated that adjustability of the length of a strut may be considered as either or both the adjustment of the assembly 18 relative to a strut (12 and/or 14), or as the adjustment of a strut (12 and/or 14) relative to the assembly 18.

The adjustable mounting of the wing assembly 18 on elongated struts 12 and 14 allows the struts to telescope or move in and out, one in opposition to the other, of the respective wing assembly 18, as will be described subsequently, to accommodate long or short legs, as one example, or long or short arms, as another example. Because the structure and function of the wing assembly is similar regardless of whether mounted to the upper strut 12 or the lower strut 14, reference will be made to only the upper strut 12 in the following description and its wing assembly 18. As well, because the structure and function of the struts 12 and 14 are identical (assuming each strut terminates in a wing assembly 18), reference to strut 12 in the following description will be construed to pertain to strut 14.

Referring to Fig. 2A, the wing assembly 18 has a wing body 20, which is preferably formed of a relatively rigid material, as for example plastic. The wing body 20 has an arcuate profile and is provided with one or more strap-retaining loops 22 for receiving the one or more adjustable straps 54 that are threaded through the loops 22 to encircle both the wing assembly 18 and a human limb, such as the leg 64 (as depicted in Fig. 1), thereby immovably securing the brace 10 to the leg 64, for example. Fig. 2B shows that the underside of the arcuate-shaped wing body 20 is provided with a generous layer of non-slip cushioning 50, both to pad the wearer's limb and to assure that the brace 10 remains in place.

Figs. 2B, 3, 4A and 4B reveal that the underside of the wing body 20 defines a unitary channel 46 that runs longitudinally down the entire length the wing body 20. While the channel 46 is generally open, splitting the cushioning 50 into two halves, a lip 48 portion of the wing body 20 overhangs the channel 46 at each of the side edges of the channel 46 down the entire longitudinal length of each side of the channel 46. The channel 46 with opposing lips 48 receives the elongated strut 12 and retains and guides the strut 12 as it telescopes in and out of the channel 46. The open nature of the channel 46 also helps to reduce the overall weight of the orthopaedic brace 10.

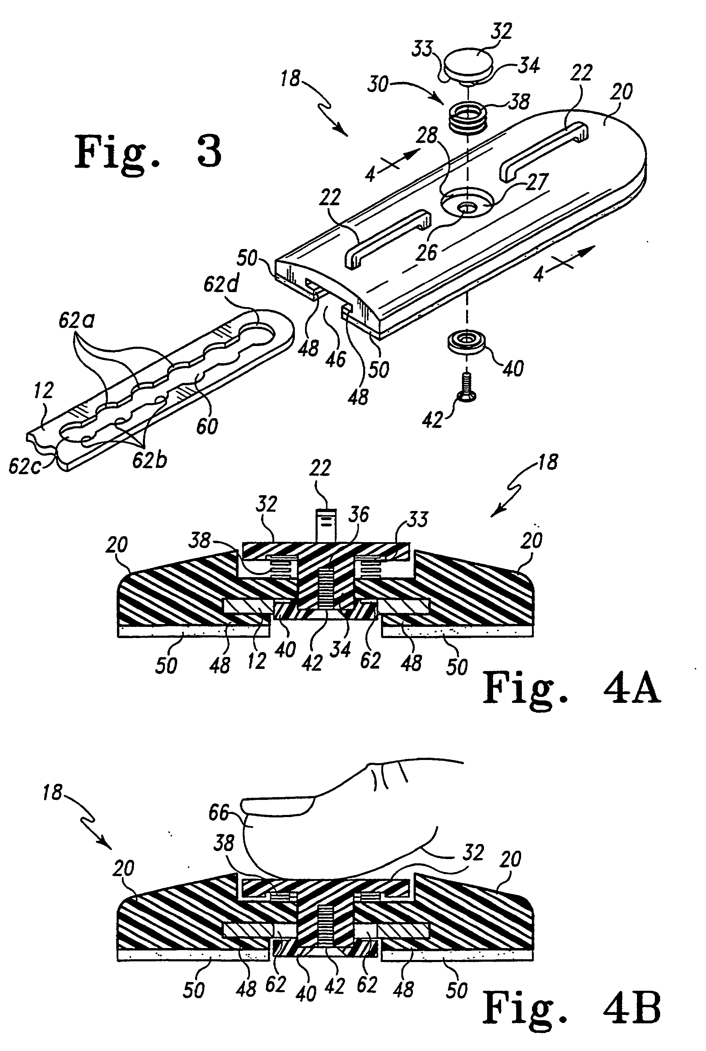

Referring to Figs. 2B and 3, the strut 12 has formed through its body a longitudinal slot 60. The length of the slot 60 may be varied depending upon the desired maximum and minimum lengths of the orthopaedic brace 10. Longitudinally spaced down each side of the slot 60 are a plurality of arcuate-shaped, stop notches generally designated 62. The notches 62 are equally divided into a plurality of notches 62a that are mirror images of, and directly across the slot 60 from, a plurality of opposing notches 62b, such that the opposing, arcuate-shaped pairs of notches 62a and 62b would define a circle if their ends were connected by an arc of constant radius equal to the distance from the centre of the slot 60 to the centre of the opposing notches. One end of the slot 60 contains an arcuate-shaped notch 62c and the other end of the slot 60 contains a mirror image arcuate-shaped notch 62d. Notches 62c and 62d are connected on each end to the outer ends of notches 62a and 62b. It should be appreciated that the notches may be shaped other than that shown.

Referring to Fig. 3, it can be seen that the wing body 20 also defines a depression or chamber 28 on the top of the body 20 which is shown as circular but can be any shape. The wing body 20 also defines an aperture 26 of smaller diameter than the chamber 28 that extends through the centre of the chamber 28 all the way to the slot 60 on the underside of the wing body 20. The chamber 28 and aperture 26 are adapted to house a positive-lock, adjustment or button assembly 30.

The adjustment assembly 30 (Fig. 3) has a generally flat pushbutton top 32 that has a cylindrical extension 34 extending downward away from and perpendicular to the top. The cylindrical extension 34 has a radius that allows it to freely travel through the aperture 26 and the slot 60 without engaging any of the notches 62a and 62b. With additional reference to Figs. 4a and 4b, a threaded aperture 36 extends down through the centre of the top 32 and the extension 34 and is adapted to receive a screw 42 from the underside of wing body 20. Fitting over the extension 34 is a biassing spring 38 of smaller diameter than the chamber 26. A retaining bushing 40, with a radius approximating that of the notches 62a, 62b, 62c and 62d, is secured to the adjustment assembly 30 (extension 34) from the underside of the wing body 20 by the screw 42, which runs through the aperture 28 into the threaded aperture 36 in the extension 34 and thus the button 32. The spring 38 is thereby secured and sandwiched between the underside 33 of the top of the button 32 and a bottom 27 of the chamber 28.

Figs. 2B and 4A show the positively locked position of the adjustment assembly 30. The spring 38 normally urges (biases) the push-button top 32 up and away from the bottom of the chamber 27 and thereby captively urges the attached bushing 40 up into the selected pair of opposing notches 62a and 62b to retain the strut 12. The bushing 40 prevents the strut 12 from longitudinally moving relative to the wing assembly 18 while the bushing 40 is within a notch.

When a finger 66 applies downward pressure on the push-button top 32, the spring 38 is compressed and pushes the connected bushing 40 down out of the opposing notches 62a and 62b. With pressure still applied, the entire wing assembly 18 can be translated up or down the slot 46 (or vice-versa) until the pressure on the button 32 is removed and the bushing (stop member) 40 re-engages one of the pair of opposing notches 62a and 62b.

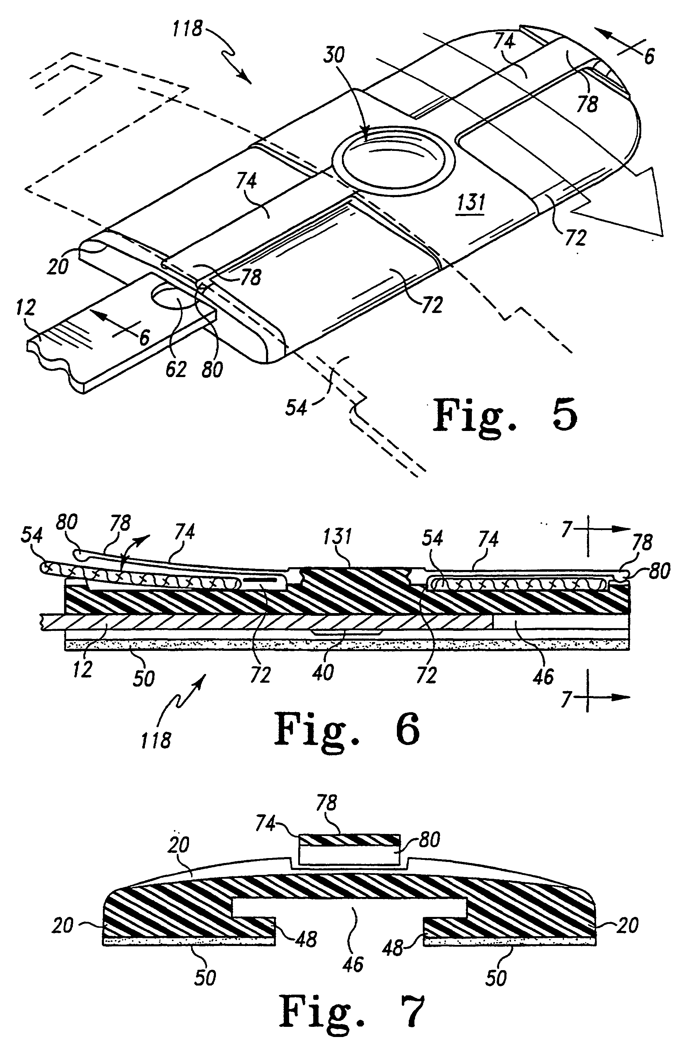

Figs. 5, 6 and 7 depict a second embodiment of a wing assembly, generally designated 118 that telescopes in the exact manner just described with respect to the wing assembly 18. The second embodiment functions the same as the wing assembly 18 with respect to the adjustment of the length of the strut 12. The wing assembly 18 is provided with at least one strap-retaining channel 72 that runs transversely across the wing member 20. A strap-retaining loop 74 extends longitudinally outward from an adjustment assembly housing 131 that retains the adjustment assembly 30 across the entire width of the channel 72 and is flush with the top of the adjustment housing 131. The loops 74 may be formed of plastic, metal, or other suitable material that is resilient enough to be repeatedly bent and still spring downward to retain the strap 54. The adjustment assembly 30 is structured and functions in like manner to the adjustment assembly 30. Features and/or functions not discussed below with respect to the wing assembly 118 should be considered to be the same as those features and/or functions with respect to the wing assembly 18 unless noted to the contrary.

This configuration gives the wing assembly 118 a lower and sleeker profile that is less likely to get caught on obstructions during use. In addition, one end 78 of the retaining loop 74 is not connected to the wing body 20. The end 78 has a nub 80 to keep the strap 54 in place (Figs. 6 and 7). The end 78 may also have a snap or other positive locking mechanism that is releasably engageable with the wing assembly 118. Referring to Fig. 6, the retaining loop 74 can be pivoted or bent up at the unconnected end 78 in order easily to slip in the strap 54. When the end 78 is released, the nub 80 ensures that the strap 54 will not slip out of the retaining channel 72. The arrow in Fig. 5 depicts where and how another strap may be placed.

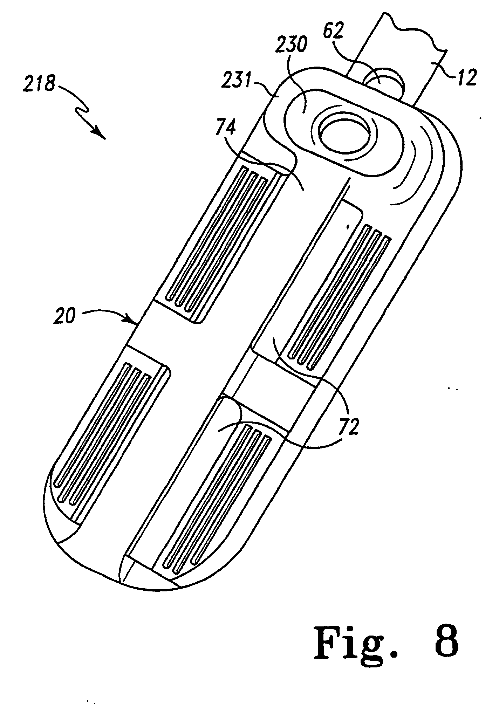

Fig. 8 depicts a third embodiment of a wing assembly, generally designated 218. This third embodiment telescopes in the exact manner described with respect to the wing assemblies 18 and 118. Other features and/or functions not discussed below with respect to the wing assembly 218 should be considered to be the same as those features and/or functions with respect to the wing assemblies 18 and 118.

The wing assembly 218 is similar in design/appearance to the wing assembly 118. The wing assembly 218 includes a body or housing 20 having a unitary retaining loop 74 that defines two channels 72 for receipt of straps (54). The adjustment assembly 230 is oval rather than round to provide easier manipulation, and is situated at an end of the body 20, proximate the strut 12. The adjustment assembly 230 is surrounded by an adjustment housing 231.

## Claims

1. An orthopaedic brace (10) comprising:
a first strut (12, 14);
a second strut (12, 14);
a hinge (16) disposed between the first and second struts (12, 14) and configured to allow movement of one of the first and second struts (12, 14) about an axis defined by the hinge (16); and
an adjustment assembly (18) disposed on one of the first and second struts (12, 14) and configured to cooperate with the one of the first and second struts (12, 14) to adjustably set an operative length of the one of the first and second struts (12, 14);
the one of the first and second struts (12, 14) including a plurality of apertures (62) corresponding to a plurality of strut length settings; **characterized in that**
the adjustment assembly (18) comprises:
a body (20) having a slot (46) configured to slidably receive the one of the first and second struts (12, 14); and
a retention assembly (30) configured to engage any one of the plurality of apertures (62) to set the length of the one of the first and second struts (12, 14), the retention assembly (30) including:
an actuator (32);
a spring (38);
a fastener (42); and
a bushing (40);
in which the fastener (42) couples the bushing (40) to the actuator (32) with the spring (38) disposed between the actuator (32) and the bushing (40).

2. A brace (10) as claimed in claim 1, in which the retention assembly (30) is normally biassed into engagement with a selective one of the plurality of apertures (62) by the spring (38).

3. A brace (10) as claimed in claim 2, in which the retention assembly (30) can be biassed into temporary disengagement from the selective one of the plurality of apertures (62).

4. A brace (10) as claimed in claim 1, in which the bushing (40) is normally biassed into engagement with a selective one of the plurality of apertures (62).

5. A brace (10) as claimed in claim 1, which includes a channel (72) configured to receive a strap (54) adapted to attach to a body part of a user.

6. A brace (10) as claimed in claim 1 further comprising:
a further adjustment assembly (18) disposed on the other of the first and second struts (12, 14) and configured to cooperate with the other of the first and second struts (12, 14) to adjustably set an operative length of the other of the first and second struts (12, 14); wherein
the other of the first and second struts (12, 14) includes a plurality of apertures (62) corresponding to a plurality of strut length settings and
the further adjustment assembly (18) comprises:
a body (20) having a slot (46) configured to slidably receive the other of the first and second struts (12, 14); and
a further retention assembly (30) configured to engage any one of the plurality of apertures (62) to set the length of the other of the first and second struts (12, 14).

7. A brace (10) as claimed in claim 6, in which the further retention assembly includes:
an actuator (32);
a spring (38);
a fastener (42); and
a bushing (40);
in which the fastener (42) couples the bushing (40) to the actuator (32) with the spring (38) disposed between the actuator (32) and the bushing (40).

8. A brace (10) as claimed in claim 7, in which the further retention assembly is normally biassed into engagement with a selective one of the plurality of apertures (62) in the other of the first and second struts (12, 14) by the second spring.

9. A brace (10) as claimed in claim 8, in which the further retention assembly (30) can be biassed into temporary disengagement from the selective one of the plurality of apertures (62) in the other of the first and second struts (12, 14)

10. A brace (10) as claimed in claim 9, in which the body (20) further comprises a channel (72) configured to receive a strap (54) adapted to attach to a body part of a user.

11. A brace (10) as claimed in claim 1 further comprising:
a wing assembly (18) disposed on one of the upper and lower struts (12,14), the wing assembly (18) including a strap retainer (22,74) configured to releasably retain a strap (54) for securing the wing assembly (18) to a body part of a user.

12. A brace (10) as claimed in claim 11, in which the strap retainer (22,74) comprises a resilient member having a first end fixed to the wing assembly (18) and a second end releasably engageable with the wing assembly (18).

13. A brace (10) as claimed in claim 12, in which the second end includes a friction engagement mechanism

14. A brace (10) as claimed in claim 1 further comprising comprising:
a strap retainer (22,74) disposed on the body (20) and configured to releasably retain a strap (54) for securing the body (20) to a body part of a user.

15. A brace as claimed in claim 14, in which the strap retainer (22,74) comprises a flexible member having a first end fixed to the body (20) and a second end releasably engagable with the body (20).

16. A brace as claimed in claim 15, in which the second end includes a friction engagement device.

## Patentansprüche

1. Orthopädische Stützvorrichtung (10), umfassend:
eine erste Abstützung (12, 14),
eine zweite Abstützung (12, 14),
ein Gelenk (16), das zwischen der ersten und zweiten Abstützung (12, 14) angeordnet und konfiguriert ist, um eine Bewegung der ersten und/oder zweiten Abstützung (12, 14) um eine Achse zu erlauben, die von dem Gelenk (16) definiert ist, und
eine Einstelleinheit (18), die auf der ersten und/oder zweiten Abstützung (12, 14) angeordnet und konfiguriert ist, um mit der ersten und/oder zweiten Abstützung (12, 14) zusammenzuwirken, um eine operative Länge der ersten und/oder zweiten Abstützung (12, 14) justierbar einzustellen,
wobei die erste und/oder die zweite Abstützung (12, 14) eine Vielzahl von Öffnungen (62) umfasst, die einer Vielzahl von Längeneinstellungen der Abstützung entsprechen, **dadurch gekennzeichnet, daß**
die Einstelleinheit (18) umfaßt:
einen Körper (20), der eine Nut (46) aufweist, die konfiguriert ist, um die erste und/oder zweite Abstützung (12, 14) gleitend aufzunehmen, und
eine Rückhalteeinheit (30), die konfiguriert ist, um in jede der Vielzahl an Öffnungen (62) einzugreifen, um die Länge der ersten und/oder zweiten Abstützung (12, 14) einzustellen, wobei die Rückhalteeinheit (30) umfaßt:
einen Aktuator (32),
eine Feder (38),
eine Befestigung (42) und
eine Buchse (40),
in welcher die Befestigung (42) die Buchse (40) an den Aktuator (32) mit der Feder (38), die zwischen dem Aktuator (32) und der Buchse (40) angeordnet ist, koppelt.

2. Stützvorrichtung (10) nach Anspruch 1, **dadurch gekennzeichnet, daß** die Rückhalteeinheit (30) normalerweise vorgespannt mittels Felder (38) in Eingriff steht mit einer aus der Vielzahl von Öffnungen (62) ausgewählten Öffnung.

3. Stützvorrichtung (10) nach Anspruch 2, **dadurch gekennzeichnet, daß** die Rückhalteeinheit (30) in ein temporäres Nicht-mehr-in-Eingriff-sein mit einer aus einer aus der Vielzahl von Öffnungen (62) ausgewählten Öffnung gebracht werden kann.

4. Stützvorrichtung (10) nach Anspruch 1, **dadurch gekennzeichnet, daß** die Buchse (40) normalerweise unter Vorspannung in Eingriff mit einer aus der Vielzahl von Öffnungen (62) ausgewählten Öffnung vorliegt.

5. Stützvorrichtung (10) nach Anspruch 1, die einen Kanal (72) umfaßt, der konfiguriert ist, um einen Gurt (54) aufzunehmen, der geeignet ist, um an einem Körperteil eines Benutzers befestigt zu werden.

6. Stützvorrichtung (10) nach Anspruch 1, ferner umfassend:
eine weitere Einstelleinheit (18), die auf der anderen Abstützung der ersten oder zweiten Abstützung (12, 14) angeordnet ist und die konfiguriert ist, um mit der anderen Abstützung der ersten und zweiten Abstützung (12, 14) zusammenzuwirken, um eine operative Länge der anderen Abstützung von erster oder zweiter Abstützung (12, 14) justierbar einzustellen, wobei
die andere Abstützung von erster oder zweiter Abstützung (12, 14) eine Vielzahl von Öffnungen (62) enthält, die einer Vielzahl von Längeneinstellungen der anderen Abstützung von erster oder zweiter Abstützung entsprechen und wobei die weitere Einstelleinheit (18) umfaßt
einen Körper (20), der eine Nut (46) aufweist, die konfiguriert ist, um gleitend die andere Abstützung von erster oder zweiter Abstützung (12, 14) aufzunehmen, und eine weitere Rückhalteeinheit (30), die konfiguriert ist, um in jede der Vielzahl an Öffnungen (62) einzugreifen, um die Länge der anderen Abstützung von erster oder zweiter Abstützung (12, 14) einzustellen.

7. Stützvorrichtung (10) nach Anspruch 6, **dadurch gekennzeichnet, daß** die weitere Rückhalteeinheit umfaßt:
einen Aktuator (32),
eine Feder (38),
eine Befestigung (42), und
eine Buchse (40),
in welcher die Befestigung (42) die Buchse (40) an den Aktuator (32) mit der Feder (38), die zwischen dem Aktuator (32) und der Buchse (40) angeordnet ist, koppelt.

8. Stützvorrichtung (10) nach Anspruch 7, **dadurch gekennzeichnet, daß** die weitere Rückhalteeinheit normalerweise vorgespannt mittels einer zweiten Feder (38) in Eingriff steht mit einer aus der Vielzahl von Öffnungen (62) ausgewählten Öffnung in der anderen Abstützung von erster oder zweiter Abstützung (12, 14).

9. Stützvorrichtung (10) nach Anspruch 8, **dadurch gekennzeichnet, daß** die weitere Rückhalteeinheit (30) in ein temporäres Nicht-mehr-in-Eingriff-sein mit einer aus der aus der Vielzahl der Öffnungen (62) ausgewählten Öffnung in der anderen Abstützung von erster oder zweiter Abstützung (12, 14) gebracht werden kann.

10. Stützvorrichtung (10) nach Anspruch 9, **dadurch gekennzeichnet, daß** der Körper (20) ferner einen Kanal (72) umfaßt, der konfiguriert ist, um einen Gurt (54) aufzunehmen, der geeignet ist, um an einem Körperteil eines Benutzers befestigt zu werden.

11. Stützvorrichtung (10) nach Anspruch 1, ferner umfassend:
eine Flügeleinheit (18), die auf einer der oberen und/oder unteren Abstützung (12, 14) angeordnet ist, wobei die Flügeleinheit (18) einen Gurtrückhalter (22, 74) umfaßt, der konfiguriert ist, um einen Gurt (54) zum Befestigen der Flügeleinheit (18) an einem Körperteil eines Benutzers freigebbar zurückzuhalten.

12. Stützvorrichtung (10) nach Anspruch 11, **dadurch gekennzeichnet, daß** der Gurtrückhalter (22, 74) ein nachgebendes Element umfaßt, von dem ein erstes Ende an der Flügeleinheit (18) befestigt ist und ein zweites Ende freigebbar mit der Flügeleinheit (18) verbindbar ist.

13. Stützvorrichtung (10) nach Anspruch 12, **dadurch gekennzeichnet, daß** das zweite Ende einen Reibungsverbindungsmechanismus umfaßt.

14. Stützvorrichtung (10) nach Anspruch 1, ferner umfassend:
einen Gurtrückhalter (22, 74), der auf dem Körper (20) angeordnet ist und der konfiguriert ist, um einen Gurt (54) zum Sichern des Körpers (20) an einem Körperteil eines Benutzers freigebbar zurückzuhalten.

15. Stützvorrichtung nach Anspruch 14, **dadurch gekennzeichnet, daß** der Gurtrückhalter (22, 74) ein flexibles Element umfaßt, von dem ein erstes Ende an dem Körper (20) befestigt ist und ein zweites Ende mit dem Körper (20) freigebbar verbindbar ist.

16. Stützvorrichtung nach Anspruch 15, **dadurch gekennzeichnet, daß** das zweite Ende eine Reibungsverbindungsvorrichtung umfaßt.

## Revendications

1. Une attelle orthopédique (10), comprenant :
une première tige (12, 14) ;
une deuxième tige (12, 14) ;
un pivot (16), disposé entre les première et deuxième tiges (12, 14) et configuré pour permettre un déplacement d'une des première et deuxième tiges (12, 14) autour d'un axe défini par le pivot (16) ; et
un ensemble d'ajustement (18), disposé sur l'une des première et deuxième tiges (12, 14) et configuré pour coopérer avec l'une des première et deuxième tiges (12, 14), afin de régler de façon ajustable une longueur fonctionnelle de l'une des première et deuxième tiges (12, 14) ;
l'une des première et deuxième tiges (12, 14) comprenant une pluralité d'ouvertures (62) correspondant à une pluralité de réglages de longueur de tige ; **caractérisée en ce que**
l'ensemble d'ajustement (18) comprend :
un corps (20) ayant une fente (46), configurée pour recevoir à coulissement l'une des première et deuxième tiges (12, 14) ; et
un ensemble de rétention (30), configuré pour venir en prise avec l'une quelconque de la pluralité des ouvertures (62) pour régler la longueur de l'une des première et deuxième tiges (12, 14), l'ensemble de rétention (30) comprenant :
un actionneur (32) ;
un ressort (38) ;
un élément de fixation (42) ; et
une douille (40) ;
dans laquelle l'élément de fixation (42) permet l'accouplement de la douille (40) à l'actionneur (32), le ressort (38) étant disposé entre l'actionneur (32) et la douille (40).

2. Une attelle (10) selon la revendication 1, dans laquelle l'ensemble de rétention (30) est normalement mis en prise avec une ouverture sélectionnée parmi la pluralité des ouvertures (62), par l'effet du ressort (68).

3. Une attelle (10) selon la revendication 2, dans laquelle l'ensemble de rétention (30) peut être mis temporairement hors de prise vis-à-vis d'une ouverture sélectionnée parmi la pluralité des ouvertures (62).

4. Une attelle (10) selon la revendication 1, dans laquelle la douille (40) est normalement mise en prise avec une ouverture sélectionnée parmi la pluralité des ouvertures (62).

5. Une attelle (10) selon la revendication 1, comprenant un canal (72), configuré pour recevoir une attache (54) adaptée pour se fixer sur une partie corporelle d'un utilisateur.

6. Une attelle (10) selon la revendication 1, comprenant en outre :
un premier ensemble d'ajustement (18), disposé sur l'autre des première et deuxième tiges (12, 14) et configuré pour coopérer avec l'une des première et deuxième tiges (12, 14), afin de régler de façon ajustable une longueur fonctionnelle de l'autre desdites première et deuxième tiges (12, 14) ; dans laquelle
l'autre desdites première et deuxième tiges (12, 14) comprend une pluralité d'ouvertures (62) correspondant à une pluralité de réglages de longueur de tige, et
l'ensemble d'ajustement (18) supplémentaire comprenant :
un corps (20), comprenant une fente (46) configurée pour loger à coulissement l'autre desdites première et deuxième tiges (12, 14) ; et
un autre ensemble de rétention (30), configuré pour venir en prise avec l'une quelconque de la pluralité des ouvertures (62) pour fixer la longueur de l'autre des première et deuxième tiges (12, 14).

7. Une attelle (10) selon la revendication 6, dans laquelle l'autre ensemble de rétention comprend :
un actionneur (32) ;
un ressort (38) ;
un élément de fixation (42) ; et
une douille (40) ;
dans laquelle l'élément de fixation (42) assure l'accouplement de la douille (40) sur l'actionneur (32), le ressort (38) étant disposé entre l'actionneur (32) et la douille (40).

8. Une attelle (10) selon la revendication 7, dans laquelle l'autre ensemble de rétention est normalement mis en prise avec une ouverture sélectionnée de la pluralité des ouvertures (62), dans l'autre de la première et de la deuxième tige (12, 14), par l'action du deuxième ressort.

9. Une attelle (10) selon la revendication 8, dans laquelle l'autre ensemble de rétention (30) peut être mis temporairement hors de prise de l'ouverture sélectionnée parmi la pluralité des ouvertures (62) situées dans l'autre des première et deuxième tiges (12, 14).

10. Une attelle (10) selon la revendication 9, dans laquelle le corps (20) comprend en outre un canal (72), configuré pour recevoir une attache (54) adaptée pour se fixer sur une partie corporelle d'un utilisateur.

11. Une attelle (10) selon la revendication 1, comprenant en outre :
un ensemble formant aile (18), disposé sur l'une des tiges supérieure et inférieure (12, 14), l'ensemble formant aile (18) comprenant un élément de retenue d'attache (22, 74), configuré pour retenir de façon désolidarisable une attache (54), afin de fixer l'ensemble formant aile (18) sur une partie corporelle d'un utilisateur.

12. Une attelle (10) selon la revendication 11, dans laquelle l'élément de retenue d'attache (22, 74) comprend un organe élastique, ayant une première extrémité, fixée sur l'ensemble formant aile (18), et une deuxième extrémité, susceptible de venir en prise de façon désolidarisable avec l'ensemble formant aile (18).

13. Une attelle (10) selon la revendication 12, dans laquelle la deuxième extrémité comprend un mécanisme de mise en prise avec friction.

14. Une attelle (10) selon la revendication 1, comprenant en outre :
un élément de retenue d'attache (22, 74), disposé sur le corps (20) et configuré pour retenir de façon désolidarisable une attache (54), afin de fixer le corps (20) sur une partie corporelle d'un utilisateur.

15. Une attelle selon la revendication 14, dans laquelle l'élément de retenue d'attache (22, 74) comprend un organe flexible, ayant une première extrémité fixée sur le corps (20) et une deuxième extrémité susceptible de venir en prise de façon désolidarisable avec le corps (20).

16. Une attelle selon la revendication 15, dans laquelle la deuxième extrémité comprend un dispositif de mise en prise avec friction.
